# EUROPEAN PATENT SPECIFICATION

(11) **EP 3 320 110 B1**
(45) Date of publication and mention of the grant of the patent: **30.12.2020**
(21) Application number: 15779009.8
(22) Date of filing: 03.09.2015
(51) Int. Cl.: C12Q 1/689, C12Q 1/6869, C12Q 1/686

(54) **16S RIBOSOMAL RNA UNIVERSAL PRIMERS AND USE THEREOF IN MICROBIOLOGICAL ANALYSIS AND DIAGNOSTICS**
UNIVERSELLE 16S-RIBOSOMALE-RNA-PRIMER UND VERWENDUNG DAVON ZUR MIKROBIOLOGISCHEN ANALYSE UND DIAGNOSE
AMORCES UNIVERSELLES D'ARN RIBOSOMIQUE 16S ET LEUR UTILISATION POUR LES ANALYSES MICROBIOLOGIQUES ET LES DIAGNOSTICS

(30) Priority: 10.07.2015 PL 41309015
(43) Date of publication of application: 16.05.2018
(73) Proprietor: Uniwersytet Jagiellonski, 31007 Krakow (PL)
(72) Inventor: GOSIEWSKI, Tomasz, PL-31-315 Kraków (PL); SALAMON, Dominika, PL-34-460 Szczawnica (PL); BULANDA, Malgorzata, 31-524 Kraków (PL); RADKOWSKI, Piotr, 30-392 Kraków (PL); LUDWIG-SLOMCZYNSKA, Agnieszka, PL-42-300 Myszków (PL); WOLKOW, Pawel, PL-31-216 Kraków (PL); SROKA-OLEKSIAK, Agnieszka, PL-30-349 Kraków (PL)
(74) Representative: BRANDPAT Patent and Trademark Attorneys Chlebicka Czyz Galazkiewicz Ziolkowski Professional Partnership
(86) International application number: PCT/IB2015/056715
(87) International publication number: WO 2017/009693

(56) References cited:
- WO-A1-2014/190394
- WEI LI ET AL: "Molecular Characterization of Skin Microbiota Between Cancer Cachexia Patients and Healthy Volunteers", MICROBIAL ECOLOGY., vol. 67, no. 3, 9 January 2014 (2014-01-09), pages 679-689, XP055252558, US ISSN: 0095-3628, DOI: 10.1007/s00248-013-0345-6
- TAKAHASHI SHUNSUKE ET AL: "Development of a Prokaryotic Universal Primer for Simultaneous Analysis of Bacteria and Archaea Using Next-Generation Sequencing", PLOS ONE, vol. 9, no. 8, August 2014 (2014-08), XP002754849,
- A. KLINDWORTH ET AL: "Evaluation of general 16S ribosomal RNA gene PCR primers for classical and next-generation sequencing-based diversity studies", NUCLEIC ACIDS RESEARCH, vol. 41, no. 1, 28 August 2012 (2012-08-28), pages e1-e1, XP055253067, GB ISSN: 0305-1048, DOI: 10.1093/nar/gks808
- DOUGLAS W FADROSH ET AL: "An improved dual-indexing approach for multiplexed 16S rRNA gene sequencing on the Illumina MiSeq platform", MICROBIOME, BIOMED CENTRAL LTD, LONDON, UK, vol. 2, no. 1, 24 February 2014 (2014-02-24), page 6, XP021179536, ISSN: 2049-2618, DOI: 10.1186/2049-2618-2-6
- E W Alm ET AL: "The oligonucleotide probe database", Applied and environmental microbiology, 1 October 1996 (1996-10-01), pages 3557-3559, XP055253101, UNITED STATES Retrieved from the Internet: URL:http://aem.asm.org/content/62/10/3557. full.pdf [retrieved on 2016-02-25]
- Jurgen Brosius ET AL: "Complete nucleotide sequence of a 16S ribosomal RNA gene from Escherichia coli", Biochemistry, 1 October 1978 (1978-10-01), pages 4801-4805, XP055252780, Retrieved from the Internet: URL:http://www.pnas.org/content/75/10/4801 .full.pdf
- S.-K. LI ET AL: "Detection and identification of plasma bacterial and viral elements in HIV/AIDS patients in comparison to healthy adults", CLINICAL MICROBIOLOGY AND INFECTION., vol. 18, no. 11, 1 November 2012 (2012-11-01), pages 1126-1133, XP055253256, United Kingdom, Switzerland ISSN: 1198-743X, DOI: 10.1111/j.1469-0691.2011.03690.x
- VASUDEVAN DINAKARAN ET AL: "Elevated Levels of Circulating DNA in Cardiovascular Disease Patients: Metagenomic Profiling of Microbiome in the Circulation", PLOS ONE, vol. 9, no. 8, 18 August 2014 (2014-08-18) , page e105221, XP055253432, DOI: 10.1371/journal.pone.0105221
- ECKER DAVID J ET AL: "Ibis T5000: a universal biosensor approach for microbiology", NATURE REVIEWS. MICROBIOLOGY,, vol. 6, no. 7, 1 July 2008 (2008-07-01), pages 553-558, XP009127531, DOI: 10.1038/NRMICRO1918
- RAMYA SRINIVASAN ET AL: "Use of 16S rRNA Gene for Identification of a Broad Range of Clinically Relevant Bacterial Pathogens", PLOS ONE, vol. 10, no. 2, 1 January 2015 (2015-01-01), page e0117617, XP55253245, DOI: 10.1371/journal.pone.0117617
- "SepsiTest(TM) Pathogen DNA Extraction and PCR Analysis Version 3.0", , 1 October 2013 (2013-10-01), pages 1-40, XP055146023, Retrieved from the Internet: URL:http://www.goffinmoleculartechnologies .com/wp-content/uploads/2014/01/SepsiTest_ V3-0_IVD_CE.pdf [retrieved on 2014-10-13]
- Michael Lustig: "SepsiTest(TM) - Culture-Independent Molecular Diagnosis of Bacterial and Fungal Infections of Joints and Bones", Trends in Molecular Diagnostics, 1 January 2012 (2012-01-01), XP055253730, Retrieved from the Internet: URL:http://www.goffinmoleculartechnologies .com/wp-content/uploads/2014/01/SepsiTest- applnote1.pdf [retrieved on 2016-02-29]
- WELLINGHAUSEN N ET AL: "Diagnosis of Bacteremia in Whole-Blood Samples by Use of a Commercial Universal 16S rRNA Gene-Based PCR and Sequence Analysis", JOURNAL OF CLINICAL MICROBIOLOGY, AMERICAN SOCIETY FOR MICROBIOLOGY, US, vol. 47, no. 9, 1 September 2009 (2009-09-01), pages 2759-2765, XP002615145, ISSN: 0095-1137, DOI: 10.1128/JCM.00567-09 [retrieved on 2009-07-01]
- C. QUAST ET AL: "The SILVA ribosomal RNA gene database project: improved data processing and web-based tools", NUCLEIC ACIDS RESEARCH, vol. 41, no. D1, 28 November 2012 (2012-11-28), pages D590-D596, XP055252806, GB ISSN: 0305-1048, DOI: 10.1093/nar/gks1219
- TOMASZ GOSIEWSKI ET AL: "A novel, nested, multiplex, real-time PCR for detection of bacteria and fungi in blood", BMC MICROBIOLOGY, BIOMED CENTRAL LTD, GB, vol. 14, no. 1, 4 June 2014 (2014-06-04), page 144, XP021187371, ISSN: 1471-2180, DOI: 10.1186/1471-2180-14-144
- ERWIN L. VAN DIJK ET AL: "Ten years of next-generation sequencing technology", TRENDS IN GENETICS., vol. 30, no. 9, 1 September 2014 (2014-09-01), pages 418-426, XP055647913, NL ISSN: 0168-9525, DOI: 10.1016/j.tig.2014.07.001
- GOSIEWSKI T ET AL: "Comprehensive detection and identification of bacterial DNA in the blood of patients with sepsis and healthy volunteers using next-generation sequencing method - the observation of DNAemia", EUROPEAN JOURNAL OF CLINICAL MICROBIOLOGY & INFECTIOUS DISEASES, SPRINGER, WIESBADEN, DE, vol. 36, no. 2, 22 October 2016 (2016-10-22), pages 329-336, XP036142355, ISSN: 0934-9723, DOI: 10.1007/S10096-016-2805-7 [retrieved on 2016-10-22]
- SCHREIBER J ET AL: "Comparison of three different commercial PCR assays for the detection of pathogens in critically ill sepsis patients", MEDIZINISCHE KLINIK, URBAN & VOGEL, MUNICH, vol. 108, no. 4, 22 March 2013 (2013-03-22), pages 311-318, XP035360210, ISSN: 2193-6218, DOI: 10.1007/S00063-013-0227-1 [retrieved on 2013-03-22]

## Description

The subject-matter of the invention is a pair of primers, and a method using said primer pair and NGS sequencing in microbiological diagnostics of blood. An innovative method of body fluids diagnosing from microbiological perspective i.e. complex analysis of bacterial profiles in the samples was developed in more detailed manner.

Microbiological diagnostics of blood is one of the most challenging diagnostics procedures. Presence of bacteria in blood (bacteriema) results frequently in sepsis, i.e. systemic inflammatory response syndrome caused by infection. Sepsis is included into one of the most challenging issues of concern of today's medicine.

Effective diagnosing the etiological factors behind systemic inflammatory response in sepsis is the key and most difficult problem deciding on treatment effectiveness and, in effect, on costs and duration of hospitalization in blood infection treatment. Determination of etiological factor allows for application of an effective and targeted antibiotic therapy. The material subject to diagnostic test is blood taken from the patient with clinical symptoms of sepsis. To this time, blood cultures on special media, preferably in automatic culture system, were considered the 'golden diagnostic standard'. The advantages of these methods include their simplicity and relatively low cost of test performance. Weak point of blood culture-based method is its time consumption, reaching even up to 5 days (until the test result is obtained) and low sensitivity, resulting in only 15-20% of cultures with microorganism growth. In addition, usually only a single bacteria species is detected, despite that their number in the patient's blood maybe higher.

To increase the probability of detecting microbiological factors in blood, multiple attempts of basing the detection on genetic methods are taken. These methods are based on nucleic acids of microorganisms in blood, such as PCR or FISH (Fluorescent In Situ Hybridization) and enable faster detection of even trace microorganisms in the samples. Sensitivity of molecular methods is much higher comparing to the culture method. In addition, earlier antibiotic therapy has no impact on test result due to no need to obtain bacteria or fungi growth on a culture medium. The only task is to detect their DNA or RNA sequence.

There are very few diagnostic sets applied in molecular diagnostics of sepsis currently available on the market, including among others SeptiFast (Roche), SeptiTest (Molzym) or VYOO (SIRS-Lab). These enable detection of the selected microorganism species or groups thereof, which allow only for confirmation of the infection rather than its exclusion. An alternative approach, consisting in detecting all possible microorganisms, is allowable - enabling both confirmation and exclusion of sepsis and, in addition, identifying the microorganisms as Gram-negative or Gram-positive bacteria and yeast and mould fungi. Development of effective molecular methods of bacteria and fungi detection in blood continues to be an issue requiring further research.

Blood culture method as well as the available molecular methods are not fully effective, despite the fact that the patients demonstrate the sepsis symptoms. Skipping the limitations of the described methods, a question about incidence rate and taxonomic diversity of bacteriemas arises.

Since recently, the microbiologists apply the sequencing technique of the new generation (NGS - New Generation Sequencing), enabling identification of all bacteria species in the sample, along with their taxonomic classification, i.e. 16S metagenomic analysis. This method is applied for detailed analysis of human and animal microbiome and testing environmental samples, for example soil or seawater samples. The NGS technology allows for elimination of the a/m difficulties.

The NGS course may be divided into three main stages. The first one is DNA isolation, the second one amplification aiming at creating the DNA library, whereas the last one is mass parallel sequencing. At present, there are several commercially sequencing platforms available on the market, among others Illumina, Roche454, SOLiD, IonTorrent and Pacific Biosciences. Common features of all these platforms include DNA isolation and single-stranded DNA library. The subsequent sequencing stages differ depending on the selected platform. Each of them has other intended use and specific technical parameters. All NGS methods are highly efficient.

In the international patent application (publ. no. WO2014/190394), the methods of identification and/or classification of microorganisms, applying one or more Single Nucleotide Polymorphisms (SNPs) in 16S ribosomal RNA (16S rRNA) in *Procaryota* and/or one or more polymorphisms in 5.8S ribosomal RNA (rRNA), 5,8S, were revealed. In addition, the probes, primers and sets used in the described methods were revealed. Also the sepsis diagnosing methods based on the reserved SNP were revealed.

The international patent application (publ. no. WO2004043236) reveals early prediction or diagnosing sepsis, enabling clinical intervention before progress of disease (i.e. at early stadium). Early diagnosing is made with the use of molecular diagnostics method by comparing biomarker expression profile of a given subject to the profiles obtained in one or more control samples.

Patent applications and descriptions such as EP 2547782, EP 2087134, EP 1978111 or EP 2009118 reveal application of PCR methods for detection of specific microorganisms based on the designed primers.

Polish patent application no. P 403 996 reveals the method of bacteria and fungi detection in biological materials sample, within which DNA contained in the sample is amplified under the PCR reaction in real time in the multiplex system, with the use of bacteria specific primers and fungi specific primers at the first stage, whereas at the second stage the formed DNA is amplified with the use of primers and probes differentiating fungi into mould and yeast fungi and bacteria into Gram positive and Gram negative. The disclosure includes oligonucleotide primers for bacteria and fungi detection using the PCR method and sets for simultaneous fungi and bacteria detection.

Polish patent application no. PL 219 490 reveals the method enabling simultaneous bacterial and fungal DNA isolation in blood. The method uses enzymatic, mechanical and thermal lysis.

The aim of the invention is supplying the new primers for amplification and the new method of diagnosing the patients with clinical sepsis symptoms. The objective adopted by the Authors includes quantitative and taxonomic identification of microorganisms in blood of patient with clinical sepsis symptoms thanks to application of NGS technique.

Despite multiple microorganism detection solutions available on the market, there is still a need of works on this issue and searches for faster and more precise methods. There has been no NGS test for specialist application in microbiological blood diagnostic or other clinical samples available on the market yet - there are only general-purpose scientific sets enabling gene sequencing or RNA or entire genomes sequencing, DNA methylation research and other tests. Such tests are manufactured by several producers, including, among others: Illumina, Roche, Life Technologies (http://www.illumina.com/technology/next-generation-sequencing.html, http://454.com/applications/index.asp, or https://www.lifetechnologies.com/pl/en/home/life-science/sequencing/next-generation-sequencing.html).

The subject-matter of the invention are primers for bacteria detection with the use of polymerase chain reaction (PCR) characterised in that it these are composed of oligonucleotides of the following sequence:
F 5' - ACGGCCNNRACTCCTAC - 3'
R 5' - TTACGGNNTGGACTACHV - 3'

Advantageously, the primers enable 16sDNA region amplification.

The other subject-matter of the invention is the method of microbiological analysis characterized in that it isolates microorganism DNA from blood with the use of enzymatic, mechanical and thermal lysis. Then DNA is amplified under the PCR reaction with the use of primers described in claim 1, followed by NGS method-based sequencing procedure for the previously amplified sequences, in line with the protocol provided by the sequencing platform producer.

Advantageously, when the tested blood derives from patients with clinical sepsis symptoms.

Advantageously, when the method is characterized in that the amplification is carried out using the ready-to-use PCR set composed of polymerase, reaction buffer, dNTPs and MgCl₂.

Advantageously, when polymerase is polymerase of low error rate in the amplified products.

Advantageously, the method comprises of the following stages: purification, labelling of the sequenced samples, post-PCR reaction product purification, determination of concentration of the purified libraries, denaturation and thinning of the internal library control and preparation of a final library.

Advantageously, the method is characterized in that the sequencing consists in simultaneous reading of sequence of the produced DNA library coding the bacterial 16SrRNA regions, and at the initial alignment of sequences to specific taxons at different taxonomic levels.

Also disclosed is a diagnostic set intended for sepsis diagnosing, characterized in that it contains the primers described under claim 1 and commercial submodules necessary to carry out the NGS process:
- MiSeq® Reagent Kit v3 (600 cycles) - catridge containing the reagents necessary to carry out the sequencing process in the sequencer
- Nextera® XT Index Kit (96 indexes, 384 samples) - a set of indexes labelling each sample with an individual code, enabling assigning of the read sequences to a given sample (patient)
- PhiX Control Kit v3 - bacteriophagic DNA constituting the sequencing control

The invention is the new method of using the existing NGS technology enabling, among others, complex research of the bacteria profiles in the samples. Until now, no potential of using this technique for blood testing in patients with sepsis has been described. Another feature distinguishing the said solution from currently available techniques is using of the designed pair of primers to perform amplification in the Nested PCR system, preceding the NGS process.

The invention enables innovative approach to the issue of micriobiological diagnostics of blood. (Scientific) sets for NGS process currently available on the market are of general use - these enable testing any type of samples (clinical or environmental). NGS may be also applied to medical diagnostics in bacteriological tests - this technique (NGS) allows for obtaining the holistic illustration of bacterial DNA presence in the sample e.g. blood sample.

The invention provides a pair of primers enabling amplification of the V3 and V4 16sDNA regions to perform PCR ampligication in the Nested system, which results in significant increase of the NGS method sensitivity.

The invention comprises the use of a new generation sequencing method. The entire process requires isolation of microorganism DNA from blood; carrying of the 16sDNA amplification to form a library and its NGS sequencing. The sequencer provides quantitative and qualitative taxonomic breakdown of all bacteria present in the sample, however with an opportunity of further bioinformatic processing to obtain more detailed information.

The core of the solution was presented in the embodiment examples described below, nature of which does not limit the scope of protection.

An experimental verification on DNA samples isolated from blood of patients with suspected sepsis (n = 42) and healthy patients (n = 13) was performed. The patients were made eligible for the study by the anesthesiologists based on presence of clinical sepsis symptoms. The blood collection procedure was carried out in compliance with the guidelines in force for blood collection for culture purposes, to confirm bacteria presence using the culture method. Difference in bacterial composition (their DNA) in both groups of patients was confirmed.

### Example 1

### Bacterial DNA isolation in blood

1. 1.5ml of full blood was added to 6mL of 0.17 M ammonium chloride
2. The samples were incubated in 37°C for 20 minutes,
3. The samples were centrifuged with a speed of 10000 rpm for 10 minutes,
4. Supernanat was removed,
5. The precipitate was suspended in 100µl of lysozyme (2mg/ml) and lysostaphin solution (0.2mg/ml) in PBS buffer,
6. The samples were conveyed to test-tubes with glass beads 700-1100 µm and mechanically disintegrated for 20 seconds at 4.0 m/s speed,
7. The samples were incubated for 30 minutes in temperature of 37°C,
8. Centrifugated with a speed of 12 000 rpm for 10 minutes,

The obtained precipitate is subject to further preparation using the commercially available DNA isolation set, in compliance with the procedural protocol provided by the manufacturer. In effect of the procedure, DNA ready for further analyses is obtained e.g. PCR reaction for bacteria detection purposes.

### Example 2

### Nested - multiplex - real time PCR for bacteria detection

The microorganism DNA amplification methodology was performed on DNA matrix isolated from human blood. Nested amplification was carried out in two separate stages marked with I and II letters. The tables below (Table 1 and 2) present the composition of reaction mixtures and thermal profiles. Stage I uses the new specific primers designed:
I amplification
F (1 ampl) ACGGCCNNRACTCCTAC
R (1 ampl) TTACGGNNTGGACTACHV

**Table 1**

| PCR | |
|---|---|
| I amplification [10 µl final volume] | |
| Water | 2.6 |
| Kapa | 5.0 |
| primer 1 (F) | 0.2 |
| primer 2 (R) | 0.2 |
| DNA | 2.0 |
| 95° - 5 minutes | |
| 95° - 15 sec. x 40 | |
| 48° - 20 sec. x 40 | |
| 72° - 30 sec. x 40 | |
| 72° - 5 minutes | |

II amplification
F
TCGTCGGCAGCGTCAGATGTGTATAAGAGACAGCCTACGGGNGGCWGCAG R GTCTCGTGGCTCGGAGATGTGTATAAGAGACAGGACTACHVGGGTATCTAATCC

**Table 2**

| NESTED PCR | |
|---|---|
| II amplification [25 µl final volume] | |
| Water | 10.5 |
| Kapa | 12.5 |
| primer 1 (F) | 0.5 |
| primer 2 (R) | 0.5 |
| Amplicon | 1.0 |
| 95° - 5 minutes | |
| 95° - 30 sec. x 40 | |
| 55° - 30 sec. x 40 | |
| 72° - 30 sec. x 40 | |
| 72° - 5 minutes | |

Amplification was carried out with ready-to-use PCR set, containing polymerase of low error rate in the amplified products. The set contains: polymerase, reaction buffer dNTPs and MgCl₂ (in final concentration of 2.5 mM)

### Example 3

### NGS procedure of amplified sequences

The sequencing procedure was carried out in the MiSeq (Illumina) apparatus, operating with software provided by the manufacturer. The sequencing process consisted in simultaneous reading of all sequences of formed DNA sequence coding the bacterial 16SrRNA regions, followed by initial alignment of sequences to specific taxons at different taxonomic levels.

The amplification processes were purified with the use of magnetic beads to eliminate free primers or starter dimmers.

### Purufication procedure:

1. A PCR plate (96 wells) with amplicons was centrifuged with a speed of 1 000 x g for 1 minute.
2. Solution with magnetic beads - AMPure XP (for DNA purification) was vortexed for app. 30 seconds, followed by introduction of 20µl to each well containing the amplicons.
3. All was mixed by pipetting up and down repeated 10 times.
4. Incubated in ambient temperature for 5 minutes.
5. The plate was placed on the magnetic mixer until transparent supernatant is obtained,
6. Leaving the plate on the magnetic mixer, supernatant is gentry removed using the pipette.
7. 200µl of newly prepared 80% ethanol was added to each sample.
8. Incubated on the magnetic mixer for 30 seconds.
9. Suparnatant was removed.
10. Steps 7-9 were repeated.
11. The plate remained on the magnetic mixer was incubated in ambient temperature for 10 minutes to dry up the beads.
12. The plate is removed from the magnetic mixer. Each sample is added with 52.5µl 10mM of Tris buffer (pH 8.5).
13. Mixed by pipetting (up and down) repeated 10 times or until the beads are entirely suspended in the Tris buffer,
14. Incubated in ambient temperature for 2 minutes.
15. The PCR plate is on the magnetic mixer until transparent supernatant is obtained.
16. 50µl of supernatant is conveyed from each well into the new 96-well PCR plate.

### Amplicons labelling (sequenced samples) - Index PCR - Procedure:

1. 5 µl from each purified amplicon into the new PCR plate was conveyed (the remaining 45µl may be frozen and re-used).
2. The primers labelled as Index 1 were placed in parallel to A-H rows of the PCR plate, whereas the primers labelled as Index 2 in parallel to 1-12 columns.
3. The reaction mixture was prepared according to the table below and introduced to the wells containing the amplicons.
4. mixed by pipetting (up and down) repeated 10 times.
5. The PCR plate was sealed with a tape contained in the set and centrifuged with a speed of 1 000 x g for 1 minute,

**Table 3. Composition of reaction mixture and reaction thermal profile.**

| Reaction mixture composition: | | Thermal conditions: |
|---|---|---|
| | | 95° - 3 minutes |
| Water | 10µl | 95° - 30 sec. x 8 |
| Kapa | 25µl | 55° - 30 sec. x 8 |
| primer (Index 1) | 5µl | 72° - 30 sec. x 8 |
| primer (Index 2) | 5µl | 72° - 5 minutes |

### Post-PCR product pufirication - Procedure:

1. The PCR plate (96 wells) containing amplicons was centrifuged with a speed of 280 x g for 1 minute.
2. The AMPure XP solution with magnetic beads was vortexed for app. 30 second, followed by introduction of 56µl to each well containing the amplicons.
3. All was mixed by pipetting up and down repeated 10 times.
4. Incubated in ambient temperature for 5 minutes.
5. The plate was placed on the magnetic mixer until transparent supernatant is obtained.
6. Leaving the plate on the magnetic mixer, supernatant is gentry removed using the pipette.
7. 200µl of newly prepared 80% ethanol was added to each sample.
8. Incubated on the magnetic mixer for 30 seconds.
9. Supernatant was removed.
10. Steps 7-9 were repeater.
11. The plate remained on the magnetic mixer was incubated in ambient temperature for 10 minutes to dry up the beads.
12. The plate is removed from the magnetic mixer. Each sample is added with 27.5µl 10mM of Tris buffer (pH 8.5).
13. Mixed by pipetting (up and down) repeated 10 times or until the beads are entirely suspended in the Tris buffer.
14. Incubated in ambient temperature for 2 minutes.
15. The PCR plate is on the magnetic mixer until transparent supernatant is obtained.
16. 25 µl of supernatant is conveyed from each well into the new 96-well PCR plate.

### Library quantification (DNA concentration determining)

Calculation of DNA concentration in the sample in nM based on fluorometric measurement using the spectrofluorometer was performed. $\frac{concentration in ng / μ l}{\frac{660 g}{mol} ∗ average library volume}$

The amplicons were thinned using 10nM of Tris buffer (pH 8.5) until concentration of 4nM was reached. From each well, 5µl of diluted DNA was sampled from each well to a single test-tube. All was mixed on the vortex.

Then, all test-tubes (libraries) were mixed together, followed by denaturization initially in NaOH thinned in the hybridization buffer and then in high temperature. Each batch contained at least 5% PhiX - a substance being the internal library control.

### Preparation:

1. The thermoblock temperature was set on 96°C.
2. A container with ice batch was prepared (2:1 ice to water ratio).

### Procedure:

1. To the prepared library a relevant quantity of newly prepared 0.2 N NaOH. (5µl 0,2N NaOH for 5µl 4nM of library).
2. All was vortexed and centrifuged with a speed of 280 x g for 1 minute.
3. Incubated in ambient temperture for DNA denaturation to single strands.
4. Relevant quantity of chilled buffer for hybridization purposes was added (990 µl of buffer per 10 µl of denaturated DNA).
Addition of buffer following the a/m recommendations for hybridization pruposes results in obtaining 20 pM of denaturated library in 1mM NaOH.
5. The test-tube with denaturated DNA was placed on ice.
6. Denaturated DNA was thinned to a desired concentration, applying the following for the provided example:

**Table 4 Principles of library thinning for obtaining of a desired concentration.**

| **Final concentration** | **2pM** | **4pM** | **6pM** | **8pM** | **10pM** |
|---|---|---|---|---|---|
| **20µl of denaturated library** | 60µl | 120µl | 180µl | 240µl | 300µl |
| **Chilled hybridization buffer** | 540µl | 480µl | 420µl | 360µl | 300µl |

7. The test-tube was mixed with thinned and denaturated DNA by turning up and down, followed by pulse centrifuging.
8. Denaturated and thinned DNA was placed on ice.

### Denaturating and thinning of the internal library control (PhiX) - Procedure:

1. PhiX was thinned to 4mM concentration: added 2µl 10nM of PhiX library to 3µl 10mM of Tris (pH 8.5) and mixed.
2. 5µl of thinned PhiX library was collected to 4mM, add up to 5µl 0.2N NaOH, all mixed.
3. Incubated in ambient temperature for 5 minutes for PhiX denaturation to single strands.
4. Relevant quantity of chilled buffer for hybridization purposes was added to test-tube containing the denaturated PhiX library to obtain 20 pM of PhiX library. To this end, add up to 10µl of denaturated PhiX control to 990µl chilled hybridization buffer.
5. Denaturated 20pM PhiX library was thinned to the same concentration as amplicon library, using Table 4.
6. The test-tube was mixed with thinned and denaturated PhiX library by turning up and down, followed by pulse centrifuging.
7. Test-tube was placed on ice.

### Preparation of final library - Procedure:

1. 30µl of denaturated and thinned PhiX library was added to 570 570µl of denaturated and thinned amplicon library.
2. All was mixed and placed on ice.
3. A sample with PhiX and amplicon library was placed in the thermoblock heated up to 96°C for 2 minutes.
4. Upon incubation the test-tube was mixed by turning up and down twice and placed immediatedly in ice batch for 5 minutes.
5. The prepared sample was placed onto appropriately labelled casette for sequencing.

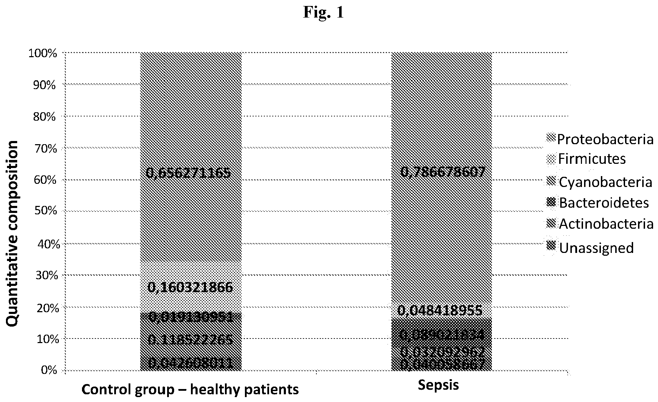

The Authors were surprised that bacterial DNA was detected also in blood of healthy patients, however their quantitative profiles were different, which was presented in Figure 1. Fig. 1 presents quantitative composition of bacterial DNA at the level of bacteria phyla in the control group and patients with sepsis.

The method limitation is no opportunity to assess whether the samples contain living bacteria cells, or their remains in a form of DNA. This may, in certain cases, hinder clinical assessment of the patient condition in context of the acquired NGS results.

### Literature

*1)* Nested-PCR as a tool for the detection and differentiation of Gram-positive and Gram-negative bacteria in patients with sepsis-septic shock. Patricia Lozano Zarain1, Genaro Lopéz-Téllez1, Rosa del Carmen Rocha-Gracial, Cuauhtémoc Romero-Lopez, Ygnacio Martínez-Laguna1, Antonio Rivera, Eduardo Brambila, African Journal of Microbiology Research Vol. 6(21), pp. 4601-4607, 9 June, 2012
*2)* The diagnosis of infectious diseases by whole genome next generation sequencing: a new era is opening, MarcLecuit and MarcEloit. Frontiersin CellularandInfectionMicrobiology, March2014|Volume4|Article25
*3)* Detection of Transient Bacteraemia following Dental Extractions by 16S rDNA Pyrosequencing: A Pilot Study Alfonso Benítez-Paéz, Maximiliano Álvarez, Pedro Belda-Ferre, Susana Rubido, Alex Mira, Inmaculada Tomá PLOS ONE | www.plosone.org 1 March 2013 | Volume 8 | Issue 3
*4)* Next-generation-sequencing-based diagnosis of sepsis directly from blood, Dr Justin O'Grady, Professor John Wain. Faculty of Medicine and Health Sciences Graduate School, School of study: Norwich Medical School
5) 16S Metagenomic Sequencing Library Preparation Preparing 16S Ribosomal RNA Gene Amplicons for the Illumina MiSeq System, Part # 15044223 Rev. B
6) Applications of Next Generation Sequencing to Blood and Marrow Transplantation, Michael Chapman, Edus H. Warren III, Catherine J. Wu, MD, Biol Blood Marrow Transplant. 2012 January ; 18(1 Suppl): S151-S160**.**
*7)* Molecular Diagnostics: Double-Digit Growth Anticipated, Executive Summary, INSIGHT PHARMA REPORTS
8) Genome-Wide Sequencing of Cellular microRNAs Identifies a Combinatorial Expression Signature Diagnostic of Sepsis. Yuqian Mal, David Vilanova, Kerem Atalar, Olivier Delfour, Jonathan Edgeworth, Marlies Ostermann, Maria Hernandez-Fuentes, Sandrine Razafimahatratra, Bernard Michot, David H. Persing, Ingrid Ziegler, et al. PLOS ONE | www.plosone.org 1 October 2013 | Volume 8 | Issue 10
9) *Detection and identification of plasma bacterial and viral elements in HIV*/*AIDS patients in comparison to healthy adults.* S.-K. Lil, R. K-K. Leung, H.-X. Guo, J.-F. Wei, J.-H. Wang, K.-T. Kwong, S.-S. Lee, C. Zhang and S. K.-W. Tsui **Clinical Microbiology and Infection** 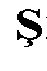 **2011 European Society of Clinical Microbiology and Infectious Diseases**

## Claims

1. Primers for bacteria detection in DNA isolated from blood of septic and bacteremia patients with the use of nested polymerase chain reaction (nested PCR) **characterized in that** these are composed of oligonucleotides of the following sequence:
F 5' - ACGGCCNNRACTCCTAC - 3'
R 5' - TTACGGNNTGGACTACHV - 3'

2. Primers according to claim 1 **characterized in that** these enable 16sDNA region amplification.

3. A method of microbiological analysis of blood **characterized in that** microorganism DNA is isolated from the blood with the use of enzymatic, mechanical and thermal lysis, followed by DNA amplification in the nested PCR reaction with the use of primers decribed in claim 1 and followed by NGS method sequencing of the previously amplified sequences, according to the protocol provided by the sequencing platform producer.

4. Method according to claim 3 **characterized in that** the tested blood derives from patients with clinical sepsis symptoms.

5. Method according to any of claims 3-4 **characterized in that** amplification is carried out with ready-to-use PCR set composed of polymerase, reaction buffer dNTPs and MgCl₂.

6. Method according to claim 5 **characterized in that** polymerase is polymerase of low error rate in amplified products.

7. Method according to claim 3 **characterized in that** it comprises of the following stages: purification, labelling of the sequenced samples, post-PCR reaction product purification, determination of concentration of the purified libraries, denaturation and thinning of the internal library control and preparation of a final library.

8. Method according to any of claims 3-7 **characterized in that** the sequencing consists in simultaneous reading of sequence of the produced DNA library coding the bacterial 16SrRNA regions, and at the initial alignment of sequences to specific taxons at different taxonomic levels.

## Patentansprüche

1. Primer zum Bakteriennachweis in DNA isoliert aus Blut von Sepsis- und Bakteriämie-Patienten mittels verschachtelter Polymerase-Kettenreaktion (nested PCR), **dadurch gekennzeichnet, dass** diese aus Oligonukleotiden mit folgender Sequenz zusammengesetzt sind:
F 5' - ACGGCCNNRACTCCTAC - 3'
R 5' - TTACGGNNTGGACTACHV - 3'

2. Primer gemäß Anspruch 1, **dadurch gekennzeichnet, dass** diese eine Amplifikation der Region 16sDNA ermöglichen.

3. Verfahren zur mikrobiologischen Analyse von Blut, **dadurch gekennzeichnet, dass** die DNA des Mikroorganismus durch enzymatische, mechanische und thermische Lyse aus dem Blut isoliert wird, gefolgt von der DNA-Amplifikation in einer verschachtelten PCR-Reaktion unter Verwendung der in Anspruch 1 beschriebenen Primer und gefolgt von der Sequenzierung der zuvor amplifizierten Sequenzen nach der NGS-Methode gemäß dem vom Hersteller der Sequenzierungsplattform bereitgestellten Protokoll.

4. Verfahren gemäß Anspruch 3, **dadurch gekennzeichnet, dass** das getestete Blut von Patienten mit klinischen Sepsis-Symptomen stammt.

5. Verfahren gemäß einem der Ansprüche 3-4, **dadurch gekennzeichnet, dass** die Amplifikation mit einem gebrauchsfertigen PCR-Set, bestehend aus Polymerase, Reaktionspuffer, dNTPs und MgCl₂, durchgeführt wird.

6. Verfahren gemäß Anspruch 5, **dadurch gekennzeichnet, dass** die Polymerase eine Polymerase mit niedriger Fehlerrate in amplifizierten Produkten ist.

7. Verfahren gemäß Anspruch 3, **dadurch gekennzeichnet, dass** es die folgenden Schritte umfasst: Aufreinigung, Markierung der sequenzierten Proben, Aufreinigung der Post-PCR-Reaktionsprodukte, Bestimmung der Konzentration der aufgereinigten Bibliotheken, Denaturierung und Verdünnung der internen Kontrolle der Bibliothek und Herstellung einer finalen Bibliothek.

8. Verfahren gemäß einem der Ansprüche 3-7, **dadurch gekennzeichnet, dass** die Sequenzierung ein gleichzeitiges Lesen der Sequenz der hergestellten DNA-Bibliothek, die bakterielle 16SrRNA-Regionen kodiert, und ein initiales Alignment der Sequenzen gegen spezifische Taxone auf verschiedenen taxonomischen Ebenen umfasst.

## Revendications

1. Amorces pour la détection de bactéries dans de l'ADN isolé à partir du sang de patients septiques et bactériémiques à l'aide d'une réaction en chaîne de la polymérase nichée (PCR nichée) **caractérisées par le fait qu'**elles sont composées d'oligonucléotides de séquence suivante :
F 5' - ACGGCCNNRACTCCTAC - 3'
R 5' - TTACGGNNTGGACTACHV - 3'.

2. Amorces selon la revendication 1, **caractérisées par le fait qu'**elles permettent une amplification de la région d'ADN 16s.

3. Méthode d'analyse microbiologique du sang **caractérisée par le fait que** l'ADN de microorganisme est isolé du sang à l'aide d'une lyse enzymatique, mécanique et thermique, suivie d'une amplification d'ADN dans la réaction de PCR nichée à l'aide des amorces décrites dans la revendication 1 et suivie de la méthode de séquençage NGS des séquences préalablement amplifiées, selon le protocole fourni par le producteur de la plateforme de séquençage.

4. Méthode selon la revendication 3, **caractérisée par le fait que** le sang testé provient de patients présentant des symptômes cliniques du sepsis.

5. Méthode selon l'une quelconque des revendications 3 ou 4, **caractérisée par le fait que** l'amplification est réalisée avec un kit PCR prêt à l'emploi composé de polymérase, de tampon de réaction dNTP et de MgCl₂.

6. Méthode selon la revendication 5, **caractérisée par le fait que** la polymérase est une polymérase à faible taux d'erreur dans les produits amplifiés.

7. Méthode selon la revendication 3, **caractérisée par le fait qu'**elle comprend les étapes suivantes : purification, marquage des échantillons séquencés, purification du produit de réaction post-PCR, détermination de la concentration des banques purifiées, dénaturation et affinement du témoin interne de la banque et préparation d'une banque finale.

8. Méthode selon l'une quelconque des revendications 3 à 7, **caractérisée par le fait que** le séquençage consiste en une lecture simultanée de la séquence de la banque d'ADN produite codant pour les régions bactériennes ARNr 16S, et à l'alignement initial de séquences sur des taxons spécifiques à différents niveaux taxonomiques.


### SEQUENCE LISTING

<110> Jagiellonian University
<120> PrimersPrimers, a method of microbiological analysis of
   biomaterial, application of NGS sequencing method in
   microbiological diagnostics and diagnostics kit
<130> PZ/3270/RW/PCT
<150> P.413090
   <151> 2015-07-10
<160> 4
<170> PatentIn version 3.5
<210> 1
   <211> 17
   <212> DNA
   <213> artificial
<220>
   <223> primer F
<220>
   <221> misc_feature
   <222> (7)..(8)
   <223> n is a, c, g, or t
<400> 1
   acggccnnra ctcctac 17
<210> 2
   <211> 18
   <212> DNA
   <213> artificial
<220>
   <223> primer R
<220>
   <221> misc_feature
   <222> (7)..(8)
   <223> n is a, c, g, or t
<400> 2
   ttacggnntg gactachv 18
<210> 3
   <211> 50
   <212> DNA
   <213> artificial
<220>
   <223> primer F II ampl
<220>
   <221> misc_feature
   <222> (42)..(42)
   <223> n is a, c, g, or t
<400> 3
   tcgtcggcag cgtcagatgt gtataagaga cagcctacgg gnggcwgcag 50
<210> 4
   <211> 55
   <212> DNA
   <213> artificial
<220>
   <223> primer R II ampl
<400> 4
   gtctcgtggg ctcggagatg tgtataagag acaggactac hvgggtatct aatcc 55